# EUROPEAN PATENT APPLICATION

(11) **EP 4 837 628 A1**
(43) Date of publication of application: **11.11.2026**
(21) Application number: 25736429.9
(22) Date of filing: 05.01.2025
(51) Int. Cl.: A61N 7/02

(54) **ULTRASONIC ABLATION DEVICE AND SYSTEM**

(30) Priority: 05.01.2024 CN 202410021220
(71) Applicant: Brattea Medtech Co., Ltd., Shanghai 201114 (CN)
(72) Inventor: GUO, Jiulin, Shanghai 200231 (CN); MAO, Zhigang, Shanghai 200231 (CN); CHEN, Hong, Shanghai 200231 (CN)
(74) Representative: Wang, Bo
(86) International application number: PCT/CN2025/070629
(87) International publication number: WO 2025/146190

(57) **Abstract**

Disclosed in the present invention are an ultrasonic ablation device and system. The ultrasonic ablation device comprises: a control part, used for automatic control of ultrasonic ablation; a catheter, connected to the control unit and at least comprises a water inlet cavity, a water outlet cavity, and a wire cavity; an ultrasonic transducer, arranged at the distal end of the catheter and connected to the control part so as to be switched between an ablation mode and a measurement mode, wherein the ultrasonic transducer is used for ultrasonic ablation in the ablation mode, and is used for temperature measurement in the measurement mode; a flexible ablation balloon, sleeved outside the ultrasonic transducer; a flexible electrode, mounted on the outer side of the balloon and used for measuring impedance; and a pressure sensor, mounted on the inner side of the balloon and used for measuring water pressure, wherein the control part is used for receiving the impedance, the water pressure, and the temperature so as to determine, on the basis of a first determination logic, whether the flexible ablation balloon is completely attached to a vessel wall; on the basis of a switching logic, controlling the ultrasonic transducer to perform mode switching; and on the basis of a second determination logic, determining whether an ablation end point is reached.

## Description

### BACKGROUND

### Technical Field

The present invention belongs to the technical field of medical instruments and relates to an ultrasound ablation device and also a corresponding ultrasound ablation system.

### Related Art

The ultrasound ablation technology refers to a situation where, using a characteristic that ultrasonic waves can penetrate human body tissues and be focused on a specific target region, energy is focused with a sufficient intensity to implement an instant high temperature at a focus region to damage tissues in the target region, showing histopathologically coagulative necrosis, thereby achieving an objective of destroying a lesion region while sparing tissues outside the lesion region. However, current ultrasound ablation devices generally cannot feed back an ablation process in real time, and ablation needs to be manually started and stopped during a procedure. This not only prolongs a procedure time, but also may cause prolonged occlusion of a blood vessel, thereby causing adverse effects in some patients. In addition, most existing ultrasound ablation balloons are rigid in design and are difficult to adapt to shapes of different blood vessels and lumens, resulting in poor attachment. This compromises an ablation effect and fails to achieve an optimal treatment effect.

### SUMMARY

A primary technical problem to be resolved in the present invention is to provide an ultrasound ablation device.

Another technical problem to be resolved in the present invention is to provide an ultrasound ablation system.

To achieve the above technical objectives, the present invention adopts the following technical solutions.

According to a first aspect of embodiments of the present invention, an ultrasound ablation device is provided, including:
a control unit, configured for automatic control of ultrasound ablation;
a catheter, where a proximal end of the catheter is connected to the control unit, and the catheter includes at least a water inflow lumen, a water outflow lumen, and a conductor wire lumen for a conductor wire to extend through;
an ultrasonic transducer, arranged at a distal end of the catheter, and electrically connected to the control unit through the conductor wire to switch between an ablation mode and a measurement mode under control of the control unit, where in the ablation mode, the ultrasonic transducer is configured to perform ultrasound ablation on a to-be-ablated area; and in the measurement mode, the ultrasonic transducer is configured to measure a temperature and tissue morphology of the to-be-ablated area;
a flexible ablation balloon, sleeved on an outer side of the ultrasonic transducer, and in communication with the water inflow lumen and the water outflow lumen;
a flexible electrode, mounted on an outer side of the flexible ablation balloon, and electrically connected to the control unit, to detect impedance; and
a pressure sensor, mounted on an inner side of the flexible ablation balloon, and electrically connected to the control unit, to detect a water pressure;
where the control unit is configured to receive the impedance and the water pressure, and determine, based on first determination logic, whether the flexible ablation balloon is completely attached to a vessel wall;
the control unit is further configured to receive the impedance, the water pressure, and the temperature and the tissue morphology of the to-be-ablated area, and control, based on switching logic, the ultrasonic transducer to switch between the ablation mode and the measurement mode; and
the control unit is further configured to receive the impedance, the water pressure, and the temperature and the tissue morphology of the to-be-ablated area and determine, based on second determination logic, whether an ablation endpoint is reached.

Preferably, the first determination logic specifically includes:
determining that the flexible ablation balloon is completely attached to the vessel wall if an impedance value reaches a preset impedance interval and the water pressure reaches a preset water pressure interval; and
determining that the flexible ablation balloon is not completely attached to the vessel wall if the impedance value does not reach the preset impedance interval and/or the water pressure does not reach the preset water pressure interval.

An ablation area is determined by an ultrasonic transducer that generates a single ultrasonic wave and calculates a distance to a wall of a blood vessel by analyzing an echo signal to obtain the ablation area.

Preferably, the switching logic specifically includes:
performing, by using a preset duration as a switching condition, a switch at an interval of the preset duration; or
receiving, by the control unit, the impedance, the water pressure, and the temperature and the tissue morphology of the to-be-ablated area, and outputting different power based on a difference between the temperature of the to-be-ablated area and a preset temperature. When an output power needs to be calculated, the ultrasonic transducer is switched to the measurement mode to measure the temperature of the to-be-ablated area, thereby enabling the control unit to calculate the output power; upon completion of calculation of the current output power, the ultrasonic transducer automatically switches to the ablation mode and operates therein for a preset duration until a single ultrasound ablation process is completed, and upon completion of the ultrasound ablation, the ultrasonic transducer automatically switches to the measurement mode to calculate the output power for next ultrasound ablation; and the ultrasonic transducer is repeatedly switched between the measurement mode and the ablation mode until the ablation endpoint is reached.

Preferably, the second determination logic specifically includes:
determining that the ablation endpoint is reached when the temperature of the to-be-ablated area measured in the measurement mode reaches the preset temperature and remains stable for the preset duration, and the water pressure and the impedance drop to a theoretical value interval.

Preferably, the control unit includes at least:
a main control module, configured to process a signal and data, and control automated ablation;
a signal generator, connected to the main control module, and configured to receive and send an ultrasonic signal;
a power amplifier, connected to the signal generator, and configured to amplify the ultrasonic signal;
a gating module, connected to the power amplifier, and configured to select a number of signal channels;
a phase voltage and current module, connected to the main control module, and configured to detect a parameter and output a matching ultrasonic frequency; and
an impedance matching module, connected to the main control module, and configured to detect matching impedance and output an ultrasonic signal at matching power.

Preferably, the ultrasonic transducer is connected to the gating module to switch between the ablation mode and the measurement mode based on a difference in numbers of signal channels;
in the ablation mode, the ultrasonic transducer converts an electrical signal into a mechanical vibration to continuously generate ultrasonic waves, and heats the to-be-ablated area within a focus range of energy transferred by sound waves; and
in the measurement mode, the ultrasonic transducer converts a pulsed electrical signal into a mechanical signal to generate a single ultrasonic wave, and calculates a tissue morphology change and a temperature change by analyzing an echo signal.

Preferably, the ultrasound ablation device further includes:
a display, connected to the control unit, and configured to display data; and
an alarm, connected to the control unit, and configured to sound an alarm in the event of a data exception.

Preferably, the catheter is further provided with a guide wire lumen, and a guide wire extends through the guide wire lumen to control a bending direction of the catheter.

Preferably, the flexible ablation balloon is made of a silica gel material, and the flexible electrode is a flexible printed circuit board.

According to a second aspect of embodiments of the present invention, an ultrasound ablation system is provided, including the ultrasound ablation device.

Compared with the prior art, the present invention has the following beneficial effects:
1. The water pressure and the impedance during a procedure and for a procedure environment are monitored in real time to ensure complete attachment to a vessel wall for a flexible ablation balloon, thereby improving attachment performance and ensuring an ablation effect.
2. The control unit controls the ultrasonic transducers to continuously switch between the ablation mode and the temperature measurement mode to implement automatic ablation, and automatically determines an ablation endpoint during ablation, thereby shortening a procedure time and reducing adverse effects on a patient during a procedure.
3. The flexible ablation balloon is made of a silica gel material, and the FPC is used for as flexible electrode, so that discomfort of a patient can be reduced while ensuring complete attachment to a vessel wall.

### BRIEF DESCRIPTION OF THE DRAWINGS

FIG. 1 is a schematic structural diagram of an ultrasound ablation device according to an embodiment of the present invention;
FIG. 2 is a block diagram of functional modules of an ultrasound ablation device according to an embodiment of the present invention;
FIG. 3 is a schematic structural diagram of a control unit according to an embodiment of the present invention; and
FIG. 4 is a schematic cross-sectional diagram of a catheter according to an embodiment of the present invention.

### DETAILED DESCRIPTION

Technical content of the present invention is described in detail below with reference to the accompanying drawings and specific embodiments.

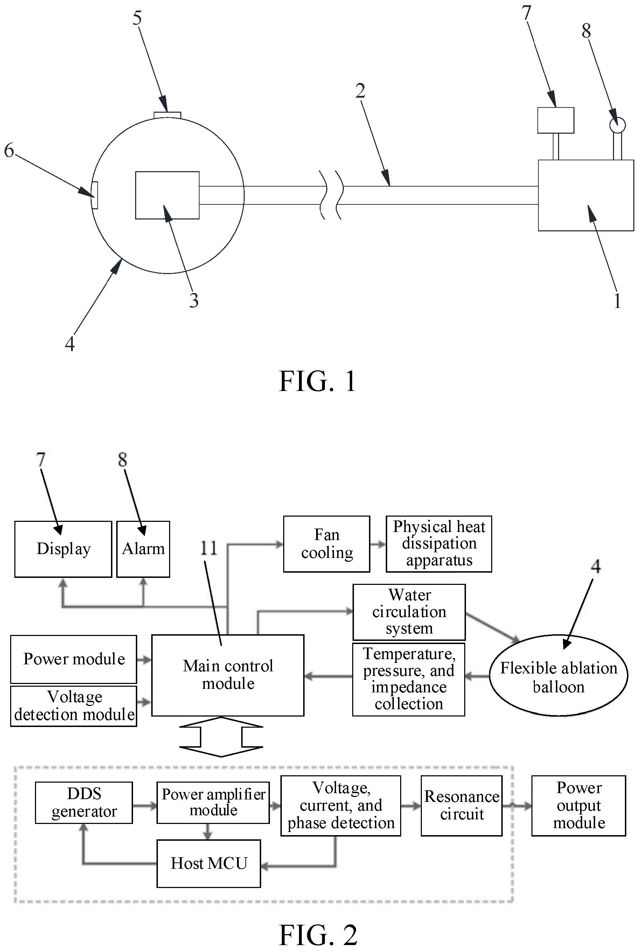

As shown in FIG. 1 and FIG. 2, an ultrasound ablation device provided in an embodiment of the present invention includes a control unit 1, a catheter 2, an ultrasonic transducer 3, a flexible ablation balloon 4, a flexible electrode 5, and a pressure sensor 6. The control unit 1 can control the ultrasonic transducer 3 to switch between an ablation mode and a measurement mode until an ablation process is completed. The flexible electrode 5 arranged on an outer side of the flexible ablation balloon 4, and the pressure sensor 6 arranged on an inner side of the flexible ablation balloon 4 are both connected to the control unit 1, so that the control unit 1 can determine whether the flexible ablation balloon 4 is completely attached to the vessel wall based on changes in impedance and a pressure.

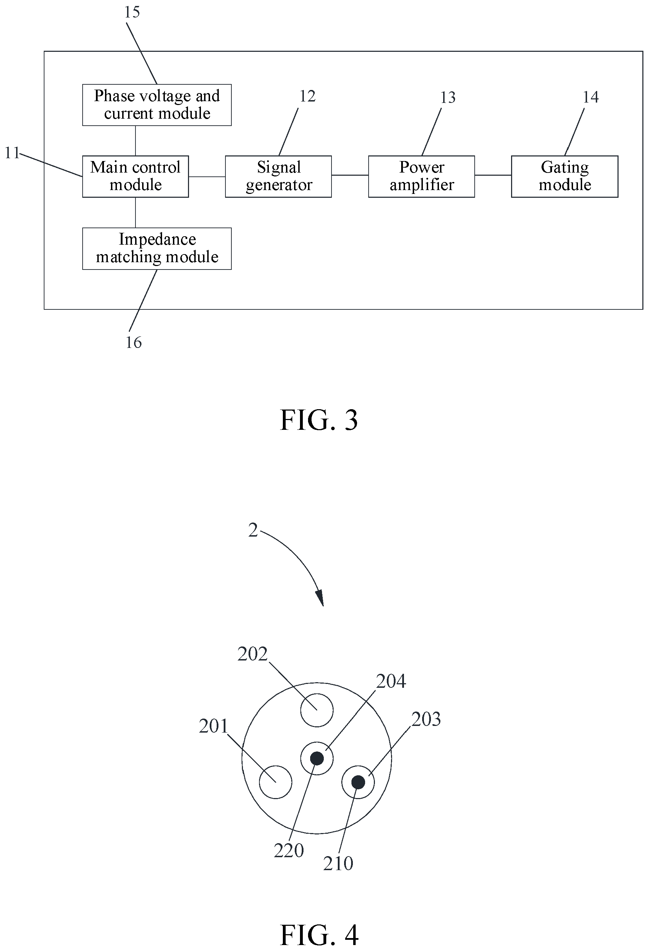

Specifically, in an embodiment of the present invention, the control unit 1 is configured for automatic control of ultrasound ablation. As shown in FIG. 3, the control unit 1 specifically includes a main control module 11, a signal generator 12, a power amplifier 13, a gating module 14, a phase voltage and current module 15, and an impedance matching module 16. The main control module 11 is configured to process a signal and data and control automated ablation. The signal generator 12 is connected to the main control module 11 to receive and send an ultrasonic signal. The power amplifier 13 is connected to the signal generator 12 to amplify the ultrasonic signal. The gating module 14 is connected to the power amplifier 13 to select a number of signal channels. The phase voltage and current module 15 is connected to the main control module 11 to detect a parameter and output a matching ultrasonic frequency. The impedance matching module 16 is connected to the main control module 11 and is configured to detect a matching impedance and output an ultrasonic signal of matching power.

A proximal end of the catheter 2 is connected to the control unit 1, and a distal end of the catheter 2 is configured to extend into a blood vessel. As shown in FIG. 4, the catheter 2 is a multi-lumen catheter, including at least a water inflow lumen 201, a water outflow lumen 202, a conductor wire lumen 203, and a guide wire lumen 204. The water inflow lumen 201 and the water outflow lumen 202 are respectively in communication with a water inlet and a water outlet of an external water supply apparatus, so as to perform water circulation. A conductor wire 210 extends through the conductor wire lumen 203, so as to electrically connect the ultrasonic transducer 3 at the distal end of the catheter 2 to the control unit 1. A guide wire 220 extends through the guide wire lumen 204 to control a bending direction of the catheter 2, thereby driving the catheter 2 to enter different blood vessel tissues. It may be understood that the catheter 2 may further be provided with another lumen according to requirements. This is not specifically limited herein.

As shown in FIG. 1 and FIG. 2, the ultrasonic transducer 3 is arranged at the distal end of the catheter 2 and is electrically connected to the gating module 14 of the control unit 1 through the conductor wire 210 to switch between the ablation mode and the measurement mode based on different signal channel numbers under control of the gating module 14. In the ablation mode, the ultrasonic transducer 3 converts an electrical signal into a mechanical vibration to continuously generate ultrasonic waves, and heats up a to-be-ablated area within a focus range for energy transferred by sound waves, thereby achieving an ultrasound ablation effect. In the measurement mode, the ultrasonic transducer 3 converts a pulsed electrical signal into a mechanical signal to generate a single ultrasonic wave, and calculates tissue morphology change and a temperature change by analyzing an echo signal, thereby measuring a temperature and tissue morphology of the to-be-ablated area.

As shown in FIG. 1 and FIG. 2, the flexible ablation balloon 4 is sleeved on an outer side of the ultrasonic transducer 3 and is in communication with the water inflow lumen 201 and the water outflow lumen 202. Therefore, water may be fed into the flexible ablation balloon 4 through an external water supply apparatus, so that the flexible ablation balloon 4 is constantly inflated until abutting against a vascular wall. Moreover, in this embodiment, the flexible electrode 5 is mounted on an outer side of the flexible ablation balloon 4 and is electrically connected to the control unit 1 to detect impedance. The pressure sensor 6 is mounted on an inner side of the flexible ablation balloon 4, and is electrically connected to the control unit 1 to detect a water pressure. Preferably, the flexible ablation balloon 4 is made of a silica gel material, and the flexible electrode 5 is a flexible printed circuit board (FPC).

As shown in FIG. 1 and FIG. 2, in the above embodiment, preferably, the ultrasound ablation device further includes a display 7 and an alarm 8. The display 7 is connected to the control unit 1 for display data. The alarm 8 is connected to the control unit 1 to sound an alarm in the event of a data exception.

In a process in which the flexible ablation balloon 4 is continuously inflated, the main control module 11 of the control unit 1 receives the impedance and the water pressure, so that it can determine, based on first determination logic, whether the flexible ablation balloon 4 is completely attached to the vessel wall, so as to ensure attachment performance of the flexible ablation balloon 4. After the flexible ablation balloon 4 is closely adhered to a vascular wall, the ultrasonic transducer 3 is switched to the ablation mode and releases ultrasonic waves to perform ultrasound ablation on the to-be-ablated area. Meanwhile, according to a preset switching logic, after first ultrasound ablation is completed, the ultrasonic transducer 3 is automatically switched to the measurement mode and perform scanning, and measurement data after the scanning is fed back to the main control module 11 for processing. The main control module 11 determines whether an ablation endpoint is reached based on a second determination logic through real-time detection of a temperature, a pressure, and impedance. The ultrasonic transducer 3 is repeatedly switched between the measurement mode and the ablation mode until the ablation endpoint is reached.

It may be understood that the external water supply apparatus maintains a current pressure during ablation to continually feed normal saline through the catheter 2, thereby cooling an interior of the flexible ablation balloon 4 while ensuring that the flexible ablation balloon 4 remains a required size. The normal saline enters the flexible ablation balloon 4 via the water inflow lumen 201 of the catheter 2 and then is discharged from the water outflow lumen 202 to a storage apparatus or flows back to a saline tank for recycling.

In an embodiment of the present invention, the first determination logic specifically includes: determining that the flexible ablation balloon is completely attached to the vessel wall if an impedance value reaches a preset impedance interval and the water pressure reaches a preset water pressure interval. In contrast, it is determined that the flexible ablation balloon is not completely attached to the vessel wall if the impedance value does not reach the preset impedance interval and/or the water pressure does not reach the preset water pressure interval.

In an embodiment of the present invention, the switching logic specifically includes performing switching at an interval of the preset duration by using a preset duration as a switching condition. Or, the control unit receives the impedance, the water pressure, and the temperature and the tissue morphology of the to-be-ablated area and outputs different power based on a difference between the temperature of the to-be-ablated area and a preset temperature. Specifically, the ultrasonic transducer first starts the measurement mode to perform a first temperature measurement on the to-be-ablated area. After the first temperature measurement is completed, temperature data is sent to a controller to calculate an output power for the first time. After the calculation of the output power for the first time is completed, the ultrasonic transducer is automatically switched to the ablation mode to release ultrasonic waves to perform first ablation (for a preset duration) on the to-be-ablated area. After the first ablation ends, the ultrasonic transducer is automatically switched to the measurement mode to perform a second temperature measurement on the to-be-ablated area to calculate the output power for a second time through a controller. After the calculation of the output power for the second time is completed, the ultrasonic transducer is switched to the ablation mode to release ultrasonic waves to perform second ablation (for the preset duration) on the to-be-ablated area, and this cycle repeats until the ablation endpoint is reached.

In an embodiment of the present invention, the second determination logic specifically includes determining that the ablation endpoint is reached when it is measured in the measurement mode that the temperature of the to-be-ablated area reaches the preset temperature and is stable for the preset duration, and the water pressure and the impedance decrease to theoretical value intervals.

The following specifically describes working steps of the ultrasound ablation device provided in embodiments of the present invention.

S1: An ultrasound ablation device performs self-checking and sends self-checking data to the main control module 11, and visualization is implemented through the display 7.

S2: A doctor sends the catheter 2 into a specified tissue of a patient, manually starts ablation, and injects normal saline into the flexible ablation balloon 4 by using an external water supply apparatus. At the beginning of the normal saline injection, a venting program for 3 to 5 seconds is performed. After the venting program is ended, the main control module 11 closes a water discharge hole and keeps injecting water through a water injection hole.

In this process, a change in a water pressure is detected through the pressure sensor 6, and a change in impedance is detected through the flexible electrode 5 to transmit the change in a water pressure and the change in impedance to the main control module 11 in real time, so that the main control module 11 determines whether the flexible ablation balloon 4 is completely attached to the vessel wall through the first determination logic. After the flexible ablation balloon is completely adhered to an inner wall of a blood vessel, ultrasound ablation is started, and the main control system 11 opens the water discharge hole and maintains a corresponding water pressure to ensure that while the flexible ablation balloon 4 maintains an adhered state, the normal saline inside the balloon is continuously circulated, thereby cooling the ultrasonic transducer 3 and the flexible ablation balloon 4.

S3: After the flexible ablation balloon 4 is completely attached to the vessel wall, the main control module 11 sends an ultrasonic signal through the signal generator 12, the signal is amplified through the power amplifier 13, and the phase voltage and current module 15 starts to work to feed back a parameter to the main control module 11 in real time for performing phase detection, and the main control module 11 adjusts a parameter of the signal generator 12 to complete phase matching.

S4: The phase-matched signal is sent to the ultrasonic transducer 3 via an appropriate number of channels selected by the gating module 14.

S5: The ultrasonic transducer 3 works cyclically to switch between the ablation mode and the temperature measurement mode based on a preset switching logic, and feeds back ultrasonic data in the measurement mode to the main control module 11, so as to determine a temperature and tissue morphology of the to-be-ablated area through the main control module 11.

S6: Data such as the temperature, the pressure, and the impedance monitored in real time, is fed back to the main control module 11 during the ablation, and the main control module 11 processes the data to determine, based on a second determination logic, whether an ablation endpoint is reached and completes an ablation procedure.

Based on the foregoing embodiments, an embodiment of the present invention further provides an ultrasound ablation system including the foregoing ultrasound ablation device. Specifically, in addition to the foregoing ultrasound ablation device, the ultrasound ablation system may further include a control handle, a control host, and the like that are connected to the foregoing ultrasound ablation device. The control handle is operated by an operator, and may be configured to perform pulling and pushing operations on a catheter 2, thereby sending the ultrasound ablation device into tissues. In addition, the control host is communicatively connected to the control unit 1 of the foregoing ultrasound ablation device (the control unit 1 herein may also be a component of the control host) to implement temperature control, energy control, and the like on the ultrasonic transducer 3, so that the ultrasonic transducer 3 can repeatedly switch between a measurement mode and an ablation mode until an ablation endpoint is reached. In addition, the ultrasound ablation system may further incorporate functions such as three-dimensional mapping, tissue density distinguishing, tissue crushing (plaque, thrombus, or the like), and ultrasonic contrast to complete an ultrasound ablation procedure in conjunction with the ultrasound ablation device.

Based on the above, the ultrasound ablation device and system provided in embodiments of the present invention have the following beneficial effects:
1. The water pressure and the impedance during a procedure and for a procedure environment are monitored in real time to ensure complete attachment to a vessel wall for the flexible ablation balloon 4, thereby improving attachment performance and ensuring an ablation effect.
2. The main control module 11 controls the ultrasonic transducer 3 to continuously switch between the ablation mode and the temperature measurement mode to implement automatic ablation, and automatically determines an ablation endpoint during ablation, thereby shortening a procedure time and reducing adverse effects on a patient during a procedure.
3. The flexible ablation balloon 4 is made of a silica gel material, and the FPC 5 is used as the flexible electrode, so that discomfort of a patient can be reduced while ensuring attachment performance.

It needs to be noted that the foregoing multiple embodiments are merely description examples. Technical solutions of embodiments may be combined, all of which fall within the protection scope of the present invention.

In addition, terms "first" and "second" are merely used for description, and cannot be understood as indicating or implying relative importance or implying a quantity of indicated technical features. Therefore, a feature defined with "first" or "second" may explicitly or implicitly include one or more features. In the description of the present invention, "a plurality of" means two or more, unless explicitly and specifically defined otherwise.

The ultrasound ablation device and system provided in the present invention are described in detail above. For a person of ordinary skill in the art, any apparent change made to the present invention without departing from the essential content of the present invention constitutes infringement of the patent right of the present invention and bears a corresponding legal responsibility.

## Claims

1. An ultrasound ablation device, comprising:
a control unit, configured for automatic control of ultrasound ablation;
a catheter, wherein a proximal end of the catheter is connected to the control unit, and the catheter comprises at least a water inflow lumen, a water outflow lumen, and a conductor wire lumen for a conductor wire to extend through;
an ultrasonic transducer, arranged at a distal end of the catheter, and electrically connected to the control unit through the conductor wire to switch between an ablation mode and a measurement mode under control of the control unit, wherein in the ablation mode, the ultrasonic transducer is configured to perform ultrasound ablation on a to-be-ablated area; and in the measurement mode, the ultrasonic transducer is configured to measure a temperature and tissue morphology of the to-be-ablated area;
a flexible ablation balloon, sleeved on an outer side of the ultrasonic transducer, and in communication with the water inflow lumen and the water outflow lumen;
a flexible electrode, mounted on an outer side of the flexible ablation balloon, and electrically connected to the control unit, to detect impedance; and
a pressure sensor, mounted on an inner side of the flexible ablation balloon, and electrically connected to the control unit, to detect a water pressure;
wherein the control unit is configured to receive the impedance and the water pressure, and determine, based on first determination logic, whether the flexible ablation balloon is completely attached to a vessel wall;
the control unit is further configured to receive the impedance, the water pressure, and the temperature and the tissue morphology of the to-be-ablated area, and control, based on switching logic, the ultrasonic transducer to switch between the ablation mode and the measurement mode; and
the control unit is further configured to receive the impedance, the water pressure, and the temperature and the tissue morphology of the to-be-ablated area and determine, based on second determination logic, whether an ablation endpoint is reached.

2. The ultrasound ablation device according to claim 1, wherein the first determination logic specifically comprises:
determining that the flexible ablation balloon is completely attached to the vessel wall if an impedance value reaches a preset impedance interval and the water pressure reaches a preset water pressure interval; and
determining that the flexible ablation balloon is not completely attached to the vessel wall if the impedance value does not reach the preset impedance interval and/or the water pressure does not reach the preset water pressure interval.

3. The ultrasound ablation device according to claim 1, wherein the switching logic specifically comprises:
performing, by using a preset duration as a switching condition, a switch at an interval of the preset duration; or
receiving, by the control unit, the impedance, the water pressure, and the temperature and the tissue morphology of the to-be-ablated area, and outputting different power based on a difference between the temperature of the to-be-ablated area and a preset temperature, wherein when an output power needs to be calculated, the ultrasonic transducer is switched to the measurement mode to measure the temperature of the to-be-ablated area, thereby enabling the control unit to calculate the output power; upon completion of calculation of the current output power, the ultrasonic transducer automatically switches to the ablation mode and operates therein for a preset duration until a single ultrasound ablation process is completed, and upon completion of the ultrasound ablation, the ultrasonic transducer automatically switches to the measurement mode to calculate the output power for next ultrasound ablation; and the ultrasonic transducer is repeatedly switched between the measurement mode and the ablation mode until the ablation endpoint is reached.

4. The ultrasound ablation device according to claim 3, wherein the second determination logic specifically comprises:
determining that the ablation endpoint is reached when the temperature of the to-be-ablated area measured in the measurement mode reaches the preset temperature and remains stable for the preset duration, and the water pressure and the impedance drop to a theoretical value interval.

5. The ultrasound ablation device according to claim 1, wherein the control unit comprises at least:
a main control module, configured to process a signal and data, and control automated ablation;
a signal generator, connected to the main control module, and configured to receive and send an ultrasonic signal;
a power amplifier, connected to the signal generator, and configured to amplify the ultrasonic signal;
a gating module, connected to the power amplifier, and configured to select a number of signal channels;
a phase voltage and current module, connected to the main control module, and configured to detect a parameter and output a matching ultrasonic frequency; and
an impedance matching module, connected to the main control module, and configured to detect matching impedance and output an ultrasonic signal at matching power.

6. The ultrasound ablation device according to claim 5, wherein:
the ultrasonic transducer is connected to the gating module to switch between the ablation mode and the measurement mode based on a difference in numbers of signal channels;
in the ablation mode, the ultrasonic transducer converts an electrical signal into a mechanical vibration to continuously generate ultrasonic waves, and heats the to-be-ablated area within a focus range of energy transferred by sound waves; and
in the measurement mode, the ultrasonic transducer converts a pulsed electrical signal into a mechanical signal to generate a single ultrasonic wave, and calculates a tissue morphology change and a temperature change by analyzing an echo signal.

7. The ultrasound ablation device according to claim 1, further comprising:
a display, connected to the control unit, and configured to display data; and
an alarm, connected to the control unit, and configured to sound an alarm in the event of a data exception.

8. The ultrasound ablation device according to claim 1, wherein:
the catheter is further provided with a guide wire lumen, and a guide wire extends through the guide wire lumen to control a bending direction of the catheter.

9. The ultrasound ablation device according to claim 1, wherein:
the flexible ablation balloon is made of a silica gel material, and the flexible electrode is a flexible printed circuit board.

10. An ultrasound ablation system, comprising the ultrasound ablation device according to any one of claims 1 to 9.
